# EUROPEAN PATENT SPECIFICATION

(11) **EP 3 554 459 B1**
(45) Date of publication and mention of the grant of the patent: **30.12.2020**
(21) Application number: 17793971.7
(22) Date of filing: 08.11.2017
(51) Int. Cl.: A61K 8/24, A61Q 11/00, A61K 8/23, A61K 8/25, A61K 8/02

(54) **ORAL CARE COMPOSITION**
MUNDPFLEGEZUSAMMENSETZUNG
COMPOSITION DE PROTECTION ORALE

(30) Priority: 14.12.2016 WO PCT/CN2016/109820; 19.01.2017 EP 17152173
(43) Date of publication of application: 23.10.2019
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); LIU, Weining, Shanghai 200335 (CN)
(74) Representative: Tansley, Sally Elizabeth
(86) International application number: PCT/EP2017/078555
(87) International publication number: WO 2018/108389

(56) References cited:
- WO-A1-2015/189041
- WO-A1-2017/005431

## Description

### Technical Field of the Invention

The present invention relates to oral care compositions such as tooth pastes, gums, and the like. In particular the present invention relates to a dual-phase oral care composition containing high levels of a water insoluble and/or slightly soluble calcium source, potassium phosphate salts and a tubule blockage enhancer that reduces tooth sensitivity. The invention also relates to the use of such compositions for treating tooth hypersensitivity and/or remineralizing and/or whitening of teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

There is a continuing need for treating tooth hypersensitivity with a more efficient approach. The present inventors have now found unexpectedly that a dual-phase oral care composition comprising high levels of a water insoluble and/or slightly soluble calcium source and a potassium phosphate salt in combination with a tubule blockage enhancer can be effective in instantly blocking dentinal tubules to reduce tooth sensitivity. Additionally, such a composition can also enhance the tooth remineralization efficacy and/or the deposition of benefit agents on tooth surfaces to further benefit teeth of an individual.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

The publication above does not describe a dual-phase oral care composition comprising a first phase comprising a water insoluble and/or slightly soluble calcium source in an amount of from 20 to 75% by weight of the first phase of the composition, a second phase comprising a potassium phosphate salt in an amount of from 30 to 75% by weight of the second phase of the composition; and wherein the first phase further comprises a tubule blockage enhancer selected from calcium dihydrogen phosphate, calcium sulfate hemihydrates or mixtures thereof, and especially such an oral care composition can treat tooth hypersensitivity and/or enhance tooth remineralization efficacy. WO2015/189041 relates to the remineralisation of the teeth.

### Tests and Definitions

### Dual-Phase

"Dual-Phase" for the purpose of the present invention means a composition having two independent phases which are physically separate.

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution are measured using Malvern Mastersizer 2000 and Malvern ZetaSizer Nano series.

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### Tubule Blockage Enhancer

"Tubule blockage enhancer" for the purpose of the present invention means a material which aids deposition of actives such as water insoluble and/or slightly soluble calcium source from the continuous phase of the composition onto dentine and/or into dentinal tubules to induce *in situ* generation of calcium phosphate that occludes the dentinal tubules and/or their exposed open ends during use of the composition. When benefit agents are present in the oral car composition, the remineralization of the calcium source around the benefit agents further helps the retention of those benefit agents on tooth surfaces by enhancing their resistance to shear force. Benefit agents as used herein means an active typically delivered to human teeth and/or the oral cavity including the gums to enhance or improve a characteristic of those dental tissues.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Water of Hydration

"Water of hydration" for the purpose of the present invention means water chemically combined with a substance in a way that it can be removed by heating without substantially changing the chemical composition of the substance. In particular, water which could only be removed when heated above 200 °C. The water loss is measured using thermo gravimetric analysis (TGA) with a Netzsch TG instrument. The TGA is conducted under an N₂ atmosphere with heating rate of 10 degree/min in the range of 30 to 900 °C.

### Substantially Free of

"Substantially free of' for the purpose of the present invention means less than 1.5%, and preferably less than 1.0%, and more preferably less than 0.75% and more preferably still less than 0.5%, and even more preferably less than 0.1 % and most preferably from 0.0 to 0.01% by weight, based on total weight of the oral care composition, including all ranges subsumed therein.

### Viscosity

Viscosity of a tooth paste or gel is the value taken at room temperature (25 °C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about". All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) a first phase comprising a water insoluble and/or slightly soluble calcium source in an amount of from 20 to 75% by weight of the first phase of the composition;
b) a second phase comprising a potassium phosphate salt in an amount of from 30 to 75% by weight of the second phase of the composition; and
wherein the first phase further comprises a tubule blockage enhancer: as defined in claim 1.

In a second aspect, the present invention is directed to a packaged oral care product comprising the oral care composition of the first aspect of this invention.

In a third aspect, the present invention is directed to a method of reducing sensitivity and/or remineralization and/or whitening of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that a dual-phase oral care composition comprising high levels of a water insoluble and/or slightly soluble calcium source and a potassium phosphate salt in combination with a tubule blockage enhancer can be effective in instantly blocking dentinal tubules to reduce tooth sensitivity. Additionally, such a composition can also enhance the tooth remineralization efficacy and/or the deposition of benefit agents on tooth surfaces to further benefit teeth of an individual.

The water insoluble and slightly soluble calcium source suitable for use in this invention is defined in clam 1.

Preferably, the calcium source is calcium silicate. In a preferred embodiment, the calcium silicate used which has low water solubility is made commercially available under the name Sorbosil CA40 by PQ Corporation. In another preferred embodiment, the calcium silicate is insoluble, present as the composite material calcium oxide-silica (CaO-SiO₂), which is described, for example, in international patent application published as WO 2008/01517(Unilever) which is hereby incorporated by reference in its entirety. For a calcium silicate composite material, the atom ratio of calcium to silicon (Ca:Si) may be from 1:30 to 5:1. The Ca:Si ratio is preferably 1:20 to 3:1, and more preferably, from 1:10 to 3:1, and most preferably, from about 1:7 to 3:1. The calcium silicate may comprise mono-calcium silicate, bi-calcium silicate, or tri-calcium silicate. The calcium silicate may be in a crystalline or amorphous state or even in a mesoporous state. In addition to calcium oxide, silica, the particles comprising the calcium silicate which is not hydrated may comprise other components, such as metal cations, anions (such as phosphate) and the like. However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In another preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 3:1, more preferably less than 1:1, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. Preferably from 10 to 100%, and especially, from 25 to 100%, and most especially, from 70% to 100% by weight of the particles comprising calcium silicate used in this invention have a particle size from 100 nm to less than 50 microns, preferably from 500 nm to 30 microns, more preferably from 1 micron to 20 microns, most preferably from 1 micron to 10 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the water insoluble and/or slightly soluble calcium source is present in an amount from 20 to 75%, more preferably from 25 to 60%, most preferably from 28 to 50%, based on the total weight of the first phase of the oral care composition and including all ranges subsumed therein.

Preferably, the first phase of the oral care composition is substantially free of phosphate sources.

The potassium phosphate salt suitable for use in this invention is able to react with the calcium source used to form hydroxyapatite *in situ.*

The potassium phosphate salt that may be used in this invention includes potassium dihydrogen phosphate, dipotassium hydrogen phosphate, tripotassium phosphate or mixtures thereof. Preferably, the potassium phosphate salt comprises potassium dihydrogen phosphate, dipotassium hydrogen phosphate or mixtures thereof. In an especially preferred embodiment, the potassium phosphate salt comprises or is a mixture of dipotassium hydrogen phosphate and potassium dihydrogen phosphate.

Typically, the potassium phosphate salt is present in an amount from 20 to 75% more preferably from 30 to 70%, most preferably from 35 to 60%, based on the total weight of the second phase of the oral care composition and including all ranges subsumed therein.

Preferably, the second phase of the composition is substantially free of calcium sources.

Preferably, the second phase of the composition is a hydrous composition. The water content in the second phase of the composition is higher than 1.5%, preferably higher than 5%, more preferably from 20 to 90% and most preferably from 40 to 60% by total weight of the second phase of the composition.

Preferably, the second phase of the composition has a pH less than 11.0, more preferably from 3.0 to 10.0, and most preferably from 5.0 to 8.0.

Other phosphate sources may also be used in the second phase of the oral care composition in addition to the potassium phosphate salt which is included in the composition. Such phosphate source includes, for example, trisodium phosphate, monosodium dihydrogen phosphate, disodium hydrogen phosphate, sodium pyrophosphate, tetrasodium pyrophosphate, sodium hexametaphosphate, mixtures thereof or the like.

The oral care composition preferably comprises the water insoluble and/or slightly soluble calcium source and the potassium phosphate salt in a weight ratio from 10:1 to 1:10, more preferably from 5:1 to 1:5, most preferably from 4:1 to 1:4.

The tubule blockage enhancer suitable for use in this invention is defined in claim 1, and is limited only to the extent that the same may be used in the mouth. In a preferred embodiment, the tubule blockage enhancer provides plenty of calcium ions in the mouth and is inexpensive and abundant. In an especially preferred embodiment, the tubule blockage enhancer is calcium salts in addition to the water insoluble and/or slightly soluble calcium source which is included in the composition.

It has been discovered that the materials used as tubule blockage enhancer in this invention is biocompatible, and undergoes rapid reaction in water and complete resorption onto the dentine and/or enamel of teeth, therefore it can be used to aid the deposition of oral care actives such as water insoluble and/or slightly soluble calcium source onto dentine and/or into dentinal tubules to induce *in situ* generation of calcium phosphate that occludes the dentinal tubules and/or their exposed open ends.

The tubule blockage enhancer is calcium dihydrogen phosphate.

Typically, the tubule blockage enhancer is present in an amount from 0.1 to 40%, more preferably from 0.5 to 30%, more preferably still from 1 to 20%, most preferably from 2 to 10%, based on the total weight of the first phase of the oral care composition and including all ranges subsumed therein.

The oral care composition preferably comprises the water insoluble and/or slightly soluble calcium source and the tubule blockage enhancer in a weight ratio from 30:1 to 1:5, more preferably from 15:1 to 1:3, most preferably from 10:1 to 1:1.

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, biomineralization agents, antibacterial agents, gum health agents, freshness agents or mixtures thereof.

For example, optical agents such as coloring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Biomineralization agents for tooth enamel remineralization may be selected from one or more of fluoride sources, biomolecules, proteinaceous materials, amorphous calcium phosphate, α-tricalcium phosphate, β-tricalcium phosphate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O). Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of antiinflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide .

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide.

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium oxide, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. The benefit agents can be present in either one of the two phases of the oral care composition. Typically, the benefit agent is present in an amount from 0.25 to 60%, and more preferably, from 0.5 to 40%, and most preferably, from 1 to 30% by total weight of the oral care composition and including all ranges subsumed therein.

When the benefit agents is incorporated into the oral care composition, the relative weight ratio of the water insoluble and/or slightly soluble calcium source to benefit agent may range from 1:10 to 30:1, preferably from 1:5 to 10:1, most preferably from 1:3 to 5:1.

The oral care composition of the present invention is found to be effective in instantly blocking dentinal tubules to reduce tooth sensitivity. Without wishing to be bound by theory the present inventors believe that this may be because the water insoluble and/or slightly soluble calcium source in the oral care composition of the present invention reacts with the potassium phosphate salt to form calcium phosphate that may have an affinity for dentine and/or enamel of teeth. The tubule blockage enhancer of the present invention may further aid the deposition of the calcium source onto dentine and/or into dentinal tubules to induce *in situ* generation of calcium phosphate that occludes the dentinal tubules and/or their exposed open ends, which enhances the tubule blockage efficacy. When benefit agents are present in the oral care composition, the remineralization of the calcium source around the benefit agents further helps the retention of those benefit agents on tooth surfaces by enhancing their resistance to shear force.

The composition of the present invention is an oral care composition and may further comprise a physiologically acceptable carrier. The carrier preferably comprises at least surfactant, thickener, humectant or a combination thereof.

Preferably the oral care composition comprises a surfactant. Preferably the composition comprises at least 0.01 % surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants. The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

The oral care composition of this invention can be used in a method of benefiting teeth of an individual comprising applying the composition to at least one surface of the teeth of an individual, said benefits includes reduced sensitivity, remineralization, whitening and combinations thereof. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

The oral care composition is a dual-phase composition comprising a first phase (calcium phase) and a second phase (phosphate phase), wherein the water insoluble and/or slightly soluble calcium source and the tubule blockage enhancer are present in the first phase and the potassium phosphate salt is present in the second phase. The two phases are physically separate from one another by being in independent phases. When a dual-phase composition is used, the first phase and the second phase should not come into contact with each other until dispensed for use.

The delivery of the two independent phases to the teeth may be simultaneous or sequentially. In a preferred embodiment, the phases are delivered simultaneously. In another preferred embodiment, the two phases are combined to form a mix prior to their application to the teeth. Typically, the weight ratio of the first phase to the second phase in this mix is from 1:3 to 10:1, more preferably from 1:2 to 7:1, most preferably from 1:1.5 to 5:1.

Preferably, the water insoluble and/or slightly calcium source is present in an amount greater than 10%, more preferably greater than 15% by total weight of the mix.

Preferably, the potassium phosphate salt is present in an amount greater than 5%, more preferably greater than 8% by total weight of the mix.

Typically, the dual-phase composition is delivered by a dual-tube having a first compartment for the first phase and a second compartment for the second phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the first phase and the sheath is the second phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases The means of delivery may involve a cotton rod, or a tray, onto which the first phase and the second phase are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The dual-phase oral care composition can be effective even when used in an individual's daily oral hygiene routine. For example, the composition may be brushed onto the teeth. The composition may, for example, be contacted with the teeth for a time period of one second to 20 hours. More preferably from 10 s to 10 hours, more preferably still from 30 s to 1 hour and most preferably from 30 s to 5 minutes. The composition may be used daily, for example for use by an individual once, twice or three times per day. In use, the two phases of the composition are mixed prior to their application to the teeth. The mixed phases are typically left on the teeth for from 3 minutes to 10 hours, more preferably from 3 minutes to 8 hours. The application may be carried out one to five times monthly.

The following examples are provided to facilitate an understanding of the present invention. The examples are not provided to limit the scope of the claims.

### Examples

### Example 1

This example demonstrates the improved blockage of dentinal tubules by using high levels of water insoluble and/or slightly soluble calcium source and potassium phosphate salts in combination with a tubule blockage enhancer. All ingredients are expressed by weight percent of the total formulation, and as level of active ingredient.

**TABLE 1**

| Ingredient | Samples | | | |
|---|---|---|---|---|
| | **1** | | **2** | |
| | First Phase | Second Phase | First Phase | Second Phase |
| Glycerol | 64.9 | -- | 69.9 | -- |
| Calcium silicate^{a} | 30.0 | -- | 30 | -- |
| Calcium dihydrogen phosphate | 5.0 | -- | -- | -- |
| Xanthan gum | 0.1 | -- | 0.1 | -- |
| Dipotassium hydrogen phosphate | -- | 30.14 | -- | 30.14 |
| Potassium dihydrogen phosphate | -- | 11.86 | -- | 11.86 |
| Water | -- | 58.00 | -- | 58.00 |

| | | | | |
|---|---|---|---|---|
| a. Commercially available calcium silicate (CaSiO₃) from P.Q.Corporation (Sorbosil CA40) | | | | |

### Methods

For Samples 1 and 2, the first phase and the second phase were mixed at a weight ratio of 3:1. To evaluate blockage efficacy of dentinal tubules, 4 g of the mix was combined with 8 mL water to make a slurry.

Human dentine disks were eroded by 37% phosphoric acid for 1 min, then they were treated with different slurries via brushing following the same protocol. The dentine disks were brushed with the slurry under a tooth brushing machine equipped with toothbrushes. The load of the tooth brushing was 170 g +/-5 g and the automatic brushing operated at a speed of 150 rpm. After brushing for 1 min, the dentine disks were soaked in slurry for 1 min. Then the dentine disks were rinsed with distilled water and placed in simulated oral fluid (SOF) under the condition of a shaking water bath at 37°C and 60.0 rpm. After soaking for about 3 to 4 hours, the dentine disks were brushed with the slurry by machine using the same procedure as in the first step. The brushing was repeated three times for one day, then the dentine disks were kept in SOF ovemight(>12 hours) in a shaking water bath at 37°C to mimic oral environment. The dentine disks were brushed for 1, 4 and 7 times.

Simulated oral fluid was made by combining the ingredients in Table 2:

**TABLE 2**

| **Ingredient** | **Amount/g** |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Results

After one brushing, SEM (Scanning Electron Microscopy) images of the dentine disks were taken. The results after one brushing were recorded as *in vitro* instant tubule blockage effect.

For each sample, four spots of the dentine disk were randomly selected and observed. From the top view of the SEM images, it can be seen that Sample 1 comprising calcium silicate and a tubule blockage enhancer showed better tubule blockage efficacy than Sample 2 comprising only the commercial calcium silicate. The measurement was repeated twice for each sample.

After 4 and 7 brushings, both samples showed good deposition on dentine surfaces and the dentinal tubules were extensively blocked.

## Claims

1. A dual-phase oral care composition comprising:
a) a first phase comprising a water insoluble and/or slightly soluble calcium source in an amount of from 20 to 75% by weight of the first phase of the composition;
b) a second phase comprising a potassium phosphate salt in an amount of from 30 to 75% by weight of the second phase of the composition; and
wherein the first phase further comprises a tubule blockage enhancer which tubule blockage enhancer is calcium dihydrogen phosphate and wherein the calcium source comprises calcium silicate, calcium carbonate, calcium aluminate, calcium oxalate, aluminium calcium silicate, calcium hydroxide, calcium glycerophosphate, calcium oxide, calcium sulfate, calcium carboxymethyl cellulose, calcium alginate, calcium salts of citric acid or mixtures thereof.

2. The oral care composition according to claim 1, wherein the potassium phosphate salt comprises potassium dihydrogen phosphate, dipotassium hydrogen phosphate, tripotassium phosphate or mixtures thereof.

3. The oral care composition according to claim 2, wherein the potassium phosphate salt comprises potassium dihydrogen phosphate, dipotassium hydrogen phosphate or mixtures thereof, preferably a mixture of potassium dihydrogen phosphate and dipotassium hydrogen phosphate.

4. The oral care composition according to any of the preceding claims, wherein the potassium phosphate salt is present in an amount from 30 to 70%, preferably from 35 to 60% by weight of the second phase of the composition.

5. The oral care composition according to any of the preceding claims, wherein the second phase of the composition has a pH from 3.0 to 10.0.

6. The oral care composition according to any of the preceding claims, wherein the calcium source is preferably calcium silicate.

7. The oral care composition according to claim 6, wherein the calcium silicate has a Ca:Si ratio of from 1:30 to 5:1, preferably from 1:20 to 3:1.

8. The oral care composition according to any of the preceding claims, wherein the calcium source is present in an amount from 25 to 60%, preferably from 28 to 50% by weight of first phase of the composition.

9. The oral care composition according to any of the preceding claims, wherein the calcium source and the tubule blockage enhancer are present in a weight ratio from 30:1 to 1:5, preferably from 15:1 to 1:3.

10. The oral care composition according to any of the preceding claims, wherein the composition further comprises a benefit agent, preferably a particulate whitening agent.

11. The oral care composition according to claim 10, wherein the particulate whitening agent is a composite particle, preferably titanium dioxide coated with calcium silicate.

12. The oral care composition according to any of the preceding claims, wherein the first phase and the second phase are physically separate prior to the use of the composition.

13. The oral care composition according to any of the preceding claims, wherein the first phase and the second phase are combined to form a mix prior to their application to the teeth, wherein the weight ratio of the first phase to the second phase in the mix is from 1:3 to 10:1, preferably from 1:2 to 7:1.

14. An oral care composition according to any one of claims 1 to 13 for use in reducing sensitivity and/or remineralizing and/or whitening of teeth.

## Patentansprüche

1. Zweiphasige Mundpflegezusammensetzung, umfassend:
a) eine erste Phase, umfassend eine wasserunlösliche und/oder schwerlösliche Calciumquelle in einer Menge von 20 bis 75 Gewichts-% der ersten Phase der Zusammensetzung;
b) eine zweite Phase, umfassend ein Kaliumphosphatsalz in einer Menge von 30 bis 75 Gewichts-% der zweiten Phase der Zusammensetzung; und
wobei die erste Phase ferner einen Röhrchenblockierungsverstärker umfasst, wobei der Röhrchenblockierungsverstärker Calciumdihydrogenphosphat ist, und wobei die Calciumquelle Calciumsilicat, Calciumcarbonat, Calciumaluminat, Calciumoxalat, Aluminiumcalciumsilicat, Calciumhydroxid, Calciumglycerophosphat, Calciumoxid, Calciumsulfat, Calciumcarboxymethylcellulose, Calciumalginat, Calciumsalze von Citronensäure oder Mischungen davon umfasst.

2. Mundpflegezusammensetzung nach Anspruch 1, wobei das Kaliumphosphatsalz Kaliumdihydrogenphosphat, Dikaliumhydrogenphosphat, Trikaliumphosphat oder Mischungen davon umfasst.

3. Mundpflegezusammensetzung nach Anspruch 2, wobei das Kaliumphosphatsalz Kaliumdihydrogenphosphat, Dikaliumhydrogenphosphat oder Mischungen davon umfasst, bevorzugt ein Gemisch aus Kaliumdihydrogenphosphat und Dikaliumhydrogenphosphat.

4. Mundpflegezusammensetzung nach irgendeinem der vorhergehenden Ansprüche, wobei das Kaliumphosphatsalz in einer Menge von 30 bis 70 Gewichts-%, bevorzugt von 35 bis 60 Gewichts-%, der zweiten Phase der Zusammensetzung vorliegt.

5. Mundpflegezusammensetzung nach irgendeinem der vorhergehenden Ansprüche, wobei die zweite Phase der Zusammensetzung einen pH von 3,0 bis 10,0 aufweist.

6. Mundpflegezusammensetzung nach irgendeinem der vorhergehenden Ansprüche, wobei die Calciumquelle bevorzugt Calciumsilicat ist.

7. Mundpflegezusammensetzung nach Anspruch 6, wobei das Calciumsilicat ein Ca:Si-Verhältnis von 1:30 bis 5:1, bevorzugt von 1:20 bis 3:1, aufweist.

8. Mundpflegezusammensetzung nach irgendeinem der vorhergehenden Ansprüche, wobei die Calciumquelle in einer Menge von 25 bis 60 Gewichts-%, bevorzugt 28 bis 50 Gewichts-%, der ersten Phase der Zusammensetzung vorliegt.

9. Mundpflegezusammensetzung nach irgendeinem der vorhergehenden Ansprüche, wobei Calciumquelle und der Röhrchenblockierungsverstärker in einem Gewichtsverhältnis von 30:1 bis 1:5, bevorzugt von 15:1 bis 1:3, vorhanden sind.

10. Mundpflegezusammensetzung nach irgendeinem der vorhergehenden Ansprüche, wobei die Zusammensetzung ferner ein Vorteil verleihendes Mittel, bevorzugt ein teilchenförmiges Weißmachermittel, umfasst.

11. Mundpflegezusammensetzung nach Anspruch 10, wobei das teilchenförmige Weißmachermittel ein Kompositteilchen ist, bevorzugt Titandioxid, beschichtet mit Calciumsilicat.

12. Mundpflegezusammensetzung nach irgendeinem der vorhergehenden Ansprüche, wobei die erste Phase und die zweite Phase vor der Verwendung der Zusammensetzung physikalisch getrennt sind.

13. Mundpflegezusammensetzung nach irgendeinem der vorhergehenden Ansprüche, wobei die erste Phase und die zweite Phase kombiniert sind, um vor ihrer Anwendung auf die Zähne eine Mischung zu bilden, wobei das Gewichtsverhältnis der ersten Phase zur zweiten Phase in der Mischung 1:3 bis 10:1, bevorzugt 1:2 bis 7:1, beträgt.

14. Mundpflegezusammensetzung nach irgendeinem der Ansprüche 1 bis 13 zur Verwendung bei der Verringerung der Empfindlichkeit und/oder zur Remineralisierung und/oder zur Aufhellung von Zähnen.

## Revendications

1. Composition pour le soin oral à phase double comprenant :
a) une première phase comprenant une source de calcium insoluble et/ou légèrement soluble dans l'eau dans une quantité de 20 à 75 % en masse de la première phase de la composition ;
b) une seconde phase comprenant un sel de phosphate de potassium dans une quantité de 30 à 75 % en masse de la seconde phase de la composition ; et
dans laquelle la première phase comprend de plus un promoteur de blocage de tubule lequel promoteur de blocage de tubule est le dihydrogénophosphate de calcium et dans laquelle la source de calcium comprend du silicate de calcium, carbonate de calcium, aluminate de calcium, oxalate de calcium, silicate d'aluminium calcium, hydroxyde de calcium, glycérophosphate de calcium, oxyde de calcium, sulfate de calcium, calcium carboxyméthylcellulose, alginate de calcium, sels de calcium d'acide citrique ou des mélanges de ceux-ci.

2. Composition pour le soin oral selon la revendication 1, dans laquelle le sel de phosphate de potassium comprend le dihydrogénophosphate de potassium, hydrogénophosphate de dipotassium, phosphate de tripotassium ou des mélanges de ceux-ci.

3. Composition pour le soin oral selon la revendication 2, dans laquelle le sel de phosphate de potassium comprend le dihydrogénophosphate de potassium, hydrogénophosphate de dipotassium ou des mélanges de ceux-ci, de préférence un mélange de dihydrogénophosphate de potassium et d'hydrogénophosphate de dipotassium.

4. Composition pour le soin oral selon l'une quelconque des revendications précédentes, dans laquelle le sel de phosphate de potassium est présent dans une quantité de 30 à 70 %, de préférence de 35 à 60 % en masse de la seconde phase de la composition.

5. Composition pour le soin oral selon l'une quelconque des revendications précédentes, dans laquelle la seconde phase de la composition présente un pH de 3,0 à 10,0.

6. Composition pour le soin oral selon l'une quelconque des revendications précédentes, dans laquelle la source de calcium est de préférence le silicate de calcium.

7. Composition pour le soin oral selon la revendication 6, dans laquelle le silicate de calcium présente un rapport Ca:Si de 1:30 à 5:1, de préférence de 1:20 à 3:1.

8. Composition pour le soin oral selon l'une quelconque des revendications précédentes, dans laquelle la source de calcium est présente dans une quantité de 25 à 60 %, de préférence de 28 à 50 % en masse de première phase de la composition.

9. Composition pour le soin oral selon l'une quelconque des revendications précédentes, dans laquelle la source de calcium et le promoteur de blocage de tubule sont présents dans un rapport massique de 30:1 à 1:5, de préférence de 15:1 à 1:3.

10. Composition pour le soin oral selon l'une quelconque des revendications précédentes, dans laquelle la composition comprend de plus un agent bénéfique, de préférence un agent blanchissant particulaire.

11. Composition pour le soin oral selon la revendication 10, dans laquelle l'agent blanchissant particulaire est une particule composite, de préférence du dioxyde de titane revêtu de silicate de calcium.

12. Composition pour le soin oral selon l'une quelconque des revendications précédentes, dans laquelle la première phase et la seconde phase sont physiquement séparées avant l'utilisation de la composition.

13. Composition pour le soin oral selon l'une quelconque des revendications précédentes, dans laquelle la première phase et la seconde phase sont combinées pour former un mélange avant leur application aux dents, dans laquelle le rapport massique de la première phase à la seconde phase dans le mélange est de 1:3 à 10:1, de préférence de 1:2 à 7:1.

14. Composition pour le soin oral selon l'une quelconque des revendications 1 à 13 pour une utilisation dans la réduction de la sensibilité et/ou la reminéralisation et/ou le blanchiment des dents.
